Europäisches Patentamt

European Patent Office

Office européen des brevets

(19)

(11) Veröffentlichungsnummer: **0 317 893**

**A2**

(12) **EUROPÄISCHE PATENTANMELDUNG**

(21) Anmeldenummer: **88119097.9**

(22) Anmeldetag: **17.11.88**

(51) Int. Cl.⁴: **A61J 7/00**

(30) Priorität: **27.11.87 DE 3740187**

(43) Veröffentlichungstag der Anmeldung:
**31.05.89 Patentblatt 89/22**

(84) Benannte Vertragsstaaten:
**AT BE CH DE ES FR GB GR IT LI LU NL SE**

(71) Anmelder: **Urquhart, John, Dr.**
**975 Hamilton Avenue**
**Palo-Alto California 94301(US)**

(72) Erfinder: **Urquhart, John, Dr.**
**975 Hamilton Avenue**
**Palo-Alto California 94301(US)**

(74) Vertreter: **Hafner, Dieter, Dr.rer.nat.,**
**Dipl.-Phys.**
**Ostendstrasse 132**
**D-8500 Nürnberg 30(DE)**

(54) **Verfahren und Vorrichtung zum Überwachen und/oder Kontrollieren und/oder Registrieren und/oder Anzeigen der Einnahme von pharmazeutischen Präparaten.**

(57) Durch das (die) neue Verfahren (Vorrichtung) sollen präzise Informationen darüber gewonnen werden, ob Präparate tatsächlich von Patienten eingenommen werden oder nicht.

Da den applizierten Präparaten jeweils wenigstens ein magnetischer Werkstoff zugesetzt ist, der auf ein von einer elektronischen Überwachungsvorrichtung erzeugtes, äußeres Magnetfeld reagierfähig ist, und der GI-Trakt des Patienten innerhalb mindestens eines vorgegebenen Zeitintervalles diesem Magnetfeld ausgesetzt wird, werden selektiv detektierbare Signale mit dem erwünschten Informationsinhalt erzeugt. Zusätzlich ist der Zeitpunkt der Arzneimittelentnahme aus der Verpackung ermittelbar und/oder eine Anwesenheits-/Identitätskontrolle des Patienten im Detektionsbereich möglich.

Eine bevorzugte Vorrichtung weist Mittel (46) zum Erfassen des Entnahmezeitpunktes des Präparats aus der Verpackung auf, Mittel (48) zur Arzneimitteleinnahmekontrolle mittels Magnetfeldes, Mittel (49) zur Anwesenheits-/Identitätskontrolle sowie eine Kontroll- und Steuereinheit (50) zum Registrieren/Anzeigen der von den Mitteln (46, 48, 49) gelieferten Ausgangssignale.

Arzneimitteleinnahmeüberwachung im privaten und klinischen Bereich.

Fig. 1

## Verfahren und Vorrichtung zum Überwachen und/oder Kontrollieren und/oder Registrieren und/oder Anzeigen der Einnahme von pharmazeutischen Präparaten.

Die Erfindung betrifft ein Verfahren zum Überwachen und/oder Kontrollieren und/oder Registrieren und/oder Anzeigen der Einnahme von pharmazeutischen Präparaten (Arzneimitteln), insbesondere in oralen oder analen Applikationsformen, seitens eines Patienten. Ferner bezieht sich die Erfindung auf eine Vorrichtung zur Durchführung dieses Verfahrens.

Der Stand der Technik:

In der medizinischen Literatur hat man sich bereits ausgiebig mit der Problematik beschäftigt, die sich bezüglich der Bereitwilligkeit, Fügsamkeit und Gewissenhaftigkeit der Patienten ergibt, die ihnen vom behandelnden Arzt verordneten Medikamente oder Arzneimittel in tatsächlicher Übereinstimmung mit der verordneten Dosierung und zu den vorgeschriebenen Zeitpunkten einzunehmen.

Aus der vorgenannten Literatur läßt sich im wesentlichen folgendes entnehmen:

1. Es gibt eine bedeutende Minorität von Patienten, welcher bei der "Selbstverwaltung" der verordneten Arzneimittel große Irrtümer unterlaufen.

2. Diese Irrtümer treten mehr oder weniger unabhängig von der Ernsthaftigkeit oder Heftigkeit der jeweiligen Erkrankung auf.

3. Die Patienten verhalten sich erfahrungsgemäß im allgemeinen so, daß sie die hinsichtlich der Dosierung bzw. der Arzneimitteleinnahme überhaupt gemachten Fehler vor ihren Ärzten verbergen oder verheimlichen; hierbei ist auch festzustellen, daß aufgrund des Verhaltens ärztlicherseits und des diagnostischen Accumens die meisten Patienten tatsächlich darin erfolgreich sind, Art und Ausmaß der ihnen unterlaufenen Dosierungsirrtümer oder -fehler vor den behandelnden Ärzten verheimlichen zu können.

4. Der im allgemeinen am häufigsten vorkommende Fehler hinsichtlich der Arzneimittel-Dosierung besteht in der Unterlassung oder dem Versäumnis der Einnahme der verordneten Arzneimittel.

5. Als weniger häufig und wahrscheinlich an die zweite Stelle tretend ist im allgemeinen ein Dosierungsfehler zu bezeichnen, welcher in dem Vergessen besteht, daß die vorgeschriebene Dosis bereits eingenommen worden ist, so daß diese nach Ablauf von etwa einer Stunde oder einer längeren Zeitspanne erneut eingenommen wird.

Aufgrund dieser Gegebenheiten ist es natürlich therapeutisch außerordentlich wünschenswert, nach Mitteln und Wegen zu suchen, die folgendes gewährleisten:

1. Überwachung der Patientenschaft hinsichtlich ihres Verhaltens bei der Einnahme und Dosierung der ihr vom Arzt verordneten Medikamente.

2. Verbesserung des Verhaltens der Patienten hinsichtlich der Dosierung und der Einnahme der Arzneimittel.

3. Minimierung der therapeutischen Konsequenzen aufgrund von Fehlern, die die Patienten bei der Dosierung machen.

4. Minimierung der Wahrscheinlichkeit, daß die behandelnden Ärzte die hinsichtlich Dosierung und Einnahme von Medikamenten auf Seiten der Patienten gemachten Fehler sodann fälschlicherweise interpretieren werden als:

a) Versagen der erwarteten Wirksamkeit des Arzneimittels;

b) Notwendigkeit für die Verordnung einer höheren Dosis als zuvor;

c) Notwendigkeit für die Verordnung eines zusätzlichen Arzneimittels;

d) Notwendigkeit für die Verschreibung eines stärkeren Arzneimittels als das erste;

e) Verschlimmerung oder Verschlechterung des Zustandes des Patienten und/oder

f) Fehldiagnose bezüglich des Zustandes des Patienten.

Die den Patienten unterlaufenen Irrtümer und Fehler hinsichtlich der "Selbstverwaltung" der ihnen verordneten Arzneimittel können natürlich zu einer unnötigen Erhöhung der Kosten, der Zeit, des Leidens sowie des Risikos führen, welchem ein Patient bei der Erzielung einer Heilung oder einer Linderung seiner Krankheit ausgesetzt ist. Eine genaue Überwachung und Kontrolle der tatsächlich eingenommenen Arzneimittel würde daher zu einer großen Verbesserung hinsichtlich der Qualität der Therapie führen, die mit den zur Verfügung stehenden Medikationen erreichbar ist.

Gemäß dem Stand der Technik gibt es bereits verschiedene Mittel und Wege, um eine "Selbstverwaltung" der z. B. zur oralen Applikation vorgesehenen Arzneimittel durch den Patienten zu überwachen, jedoch sind diese bekannten Mittel und Wege mit wenigen Ausnahmen sämtlich als indirekt zu bezeichnen, und zwar insofern, als sie nicht den tatsächlichen Beweis erbringen, daß ein verordnetes Medikament in der vorgeschriebenen Dosierung tatsächlich eingenommen worden ist. Darüber hinaus können im Falle, daß verschiedene Stärken der Dosierung für den Patienten verfügbar sind, diese bekannten Maßnahmen nicht aufzeigen, in welcher Dosierungsstärke das Medikament vom Patienten eingenommen worden ist.

Die derzeit verfügbaren Methoden und Techniken zur Überwachung der oralen oder analen Arzneimittel-Einnahme können in zwei Kategorien eingeordnet werden:

a) Die kontinuierliche Kategorie;
b) die intermittierende Kategorie.

In der kontinuierlichen Kategorie gibt es bereits eine Anzahl von Anwendungen von elektrischen und mechanischen Mitteln, die zu der Feststellung oder zum Ergreifen geeigneter Maßnahmen dienen, wenn z. B. eine orale Applikationsform, z. B. Pille, Tablette oder Kapsel, aus ihrer zugehörigen Packung entnommen worden ist. Hierbei ist unter "geeigneten Maßnahmen" folgendes zu verstehen:

Die Aufzeichnung der Zeit, die Auslösung eines Alarmes oder das Aussenden eines Signals zu einem etwa entfernt angeordneten Überwachungsgerät oder zu einer sich entfernt aufhaltenden dritten Person.

Durch keine der vorstehend erläuterten Methoden kann jedoch tatsächlich festgestellt oder bestätigt werden, in welcher Stärke oder Dosierung ein Medikament, wenn überhaupt, von dem Patienten eingenommen worden ist.

Ferner sind in der intermittierenden Kategorie verschiedene Maßnahmen zur Feststellung oder Ermittlung von Dosierungsfehlern bekannt. Solche Maßnahmen bestehen beispielsweise darin, intermittierend Proben vom Blut, vom Urin oder von anderen Körperflüssigkeiten des Patienten zu entnehmen und diese Proben zu analysieren, um auf direktem Wege entweder das Arzneimittel selbst oder einen Metabolit des Arzneimittels oder ganz bestimmte Chemikalien zu ermitteln, deren Vorhandensein eindeutig beweisen, daß das betreffende Arzneimittel von dem Patienten eingenommen worden ist.

Mit Hilfe derartiger Maßnahmen und Methoden läßt sich feststellen, daß das betreffende Arzneimittel tatsächlich eingenommen worden ist und zwar zu irgendeiner gewissen Zeit und in irgendeiner gewissen Dosis, es läßt sich aber damit nicht genau spezifizieren, "wann" das Medikament oder "wieviel" von dem Medikament eingenommen worden ist. Somit sind diese bekannten Methoden mit den Nachteilen behaftet, daß sie sich nicht zu einer Extrapolation von gelegentlichen Messungen oder Ermittlungen zu einer kontinuierlichen Aufzeichnung darüber eignen, wann das Präparat oder Arzneimittel tatsächlich eingenommen worden ist.

Eine weitere bekannte Methode zur Feststellung, ob ein Patient vergessen hat, ein Medikament in der vorgeschriebenen Dosis einzunehmen, besteht darin, das Öffnen und Schließen der Arzneimittelverpackung zu überwachen oder die Entnahme einer Medikamentendosis aus der Verpackung durch den Patienten zu überwachen.

Vorrichtungen zum Feststellen und Dokumentieren des jeweiligen genauen Zeitpunktes der Entnahme eines Medikamentes aus der zugehörigen Verpackung sind bereits bekannt durch: DE-PS 33 35 301, die DE-OSen 35 04 431 und 35 30 356, EP 0 191 168 A 2, US-PSen 4 526 474 und 4 660 991. Jedoch kann mit Hilfe dieser bekannten Methoden auch kein Beweis geführt werden, daß die vorgeschriebene Dosis von dem Patienten tatsächlich eingenommen worden ist, sondern, damit läßt sich nur feststellen, ob die Arzneimittelverpackung geöffnet oder nicht geöffnet worden war und gegebenenfalls, wann sie geöffnet worden war.

Mit Rücksicht auf die im Vorangehenden erläuterten Probleme und Schwierigkeiten hinsichtlich der Überwachung der "Selbstverwaltung" von Arzneimitteln durch die Patienten, und insbesondere hinsichtlich der Kontinuität einer derartigen Überwachung liegt nun der vorliegenden Erfindung die Aufgabe zugrunde, ein Verfahren gemäß dem Oberbegriff des Patentanspruchs 1 anzugeben, durch das eine sichere, eindeutige, registrierbare und/oder anzeigbare Information darüber gewonnen wird, ob das vom Arzt verordnete Präparat oder Arzneimittel tatsächlich vom Patienten eingenommen worden ist, evtl. ergänzt durch die zusätzlichen Informationen, in welcher Dosis und/oder zu welchem Zeitpunkt die Einnahme erfolgt ist, und evtl. auch weiterhin noch ergänzt durch eine Information bezüglich der Lokalisierbarkeit des eingenommenen Präparates im Bereich des Gastro-Intestinal-Traktes eines Patienten.

Diese Aufgabe wird erfindungsgemäß bei einem Verfahren zum Überwachen und/oder Kontrollieren und/oder Registrieren und/oder Anzeigen der Einnahme von pharmazeutischen Präparaten, insbesondere in oralen oder analen Applikationsformen, seitens eines Patienten, erfindungsgemäß im wesentlichen durch die Kombination der folgenden Maßnahmen gelöst:

a) Es wird dem Patienten ein Präparat in einer vorgegebenen Dosis verabreicht, wobei diesem Präparat, insbesondere dem (den) Wirkstoff-(en) des Präparats wenigstens ein magnetischer Werkstoff zugesetzt ist, der auf ein von außen einwirkendes Magnetfeld einer elektronischen Überwachungsvorrichtung reagierfähig ist;

b) es wird ein äußeres Magnetfeld erzeugt, dem der Gastro- Intestinal-Trakt des jeweiligen Patienten innerhalb mindestens eines vorgegebenen Zeitintervalles ausgesetzt wird; und

c) es werden diejenigen Störungen, die in dem erzeugten Magnetfeld durch einen oder mehrere gegebenenfalls im Gastro-Intestinal-Trakt des Patienten vorhandene, aus dem magnetischen Werkstoff bestehende Körper in deren magnetisch detektierbarem Zustand induziert werden, empfangen und/oder detektiert und/oder angezeigt, und zwar in der Weise, daß durch die elektronische Überwachungsvorrichtung selektiv detektierbare Signale erzeugt werden, die zur Lieferung einer eindeutigen Information, ob das Präparat von dem Patienten eingenommen ist oder nicht, und/oder zur Lokalisierung des Präparates bzw. des magnetischen Werkstoffes im Bereich des Gastro-Intestinal-Traktes des Patienten weiter verarbeitet werden.

Gemäß einer Weiterbildung des erfindungsgemäßen Verfahrens wird zusätzlich jeweils der Zeitpunkt, zu dem das Präparat aus der zugehörigen Verpackung entnommen wird, ermittelt und/oder registriert, und/oder angezeigt, insbesondere durch entsprechende Signal-Erzeugung und - Verarbeitung.

Weitere Ausgestaltungen des erfindungsgemäßen Verfahrens ergeben sich auch aus den Ansprüchen 3 - 6.

Eine weitere, der vorliegenden Erfindung zugrunde liegende Aufgabe besteht darin, eine Vorrichtung zur Durchführung des Verfahrens nach Anspruch 1 zu schaffen. Gemäß der Erfindung ist diese Vorrichtung als eine elektronische Überwachungsvorrichtung ausgebildet, die im wesentlichen die folgenden Bestandteile aufweist:

a) Mittel zum Erzeugen eines äußeren Magnetfeldes, dem der Gastro-Intestinal-Trakt des jeweiligen Patienten innerhalb mindestens eines vorgegebenen Zeitintervalles ausgesetzt wird;

b) Mittel zum Empfangen und/oder selektiven Detektieren von Störungen, die in dem erzeugten Magnetfeld durch einen oder mehrere gegebenenfalls im Gastro-Intestinal-Trakt des Patienten vorhandene, aus einem magnetischen, auf das von außen einwirkende Magnetfeld reagierfähigen Werkstoff bestehende Körper in derem magnetisch detektierbaren Zustand induziert werden;

c) Mittel zum Erzeugen von selektiv detektierbaren Signalen aufgrund der empfangenen und/oder selektiv detektierten Störungen in dem erzeugten Magnetfeld;

d) Mittel zur Weiterverarbeitung der gemäß c) erzeugten Signale zur Lieferung einer eindeutigen Information, ob das Präparat von dem Patienten eingenommen ist oder nicht und/oder zur Lokalisierung des Präparats bzw. des magnetischen Werkstoffes im Bereich des Gastro-Intestinal-Traktes des Patienten, in Form entsprechender Ausgangssignale und

e) Mittel zum Registrieren und/oder Anzeigen der gemäß d) gebildeten Ausgangssignale.

Weitere vorteilhafte Ausgestaltungen dieser erfindungsgemäßen Vorrichtung ergeben sich aus den Ansprüchen 8 -21.

Zur näheren Erläuterung der Erfindung, ihrer weiteren Merkmale und Vorteile dient die beigefügte Beschreibung von Ausführungsbeispielen in Verbindung mit den Zeichnungen.

Dabei zeigt:

Fig. 1 einen Schnitt durch ein pharmazeutisches Präparat oder Medikament in der Form einer Pille;

Fig. 2 einen Schnitt durch ein Medikament in der Form einer Kapsel;

Fig. 3 ein Blockschaltbild einer elektronischen Überwachungsvorrichtung in Übereinstimmung mit der vorliegenden Erfindung;

Fig. 4 eine Hystereseschleife zur Erläuterung der magnetischen Eigenschaften des den pharmazeutischen Präparaten zugesetzten magnetischen Werkstoffes;

Fig. 5 schematisch in Seitenansicht eine Ausführung einer elektronischen Überwachungsvorrichtung in Übereinstimmung mit der vorliegenden Erfindung;

Fig. 6 schematisch in Seitenansicht eine weitere Ausführungsart einer elektronischen Überwachungs vorrichtung in Übereinstimmung mit der vorliegenden Erfindung;
und

Fig. 7 schematisch ein Blockschaltbild eines Überwachungssystems in Verbindung mit einer zentralen Kontroll- und Steuereinheit.

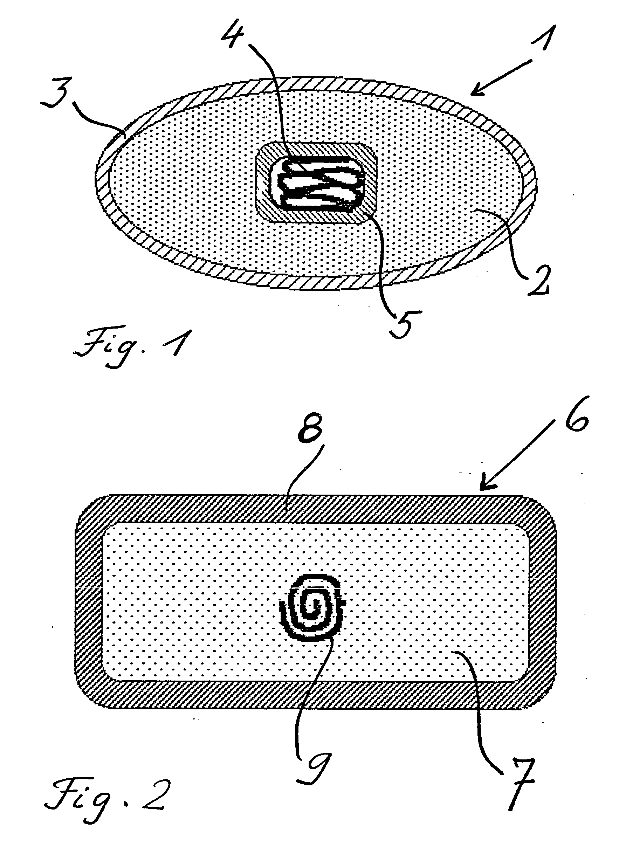

Die in den Fig. 1 und 2 dargestellten Ausführungsbeispiele von pharmazeutischen Präparaten sind jeweils in vergrößerter Darstellung gezeichnet, um das Verständnis der vorliegenden Erfindung zu erleichtern.

Fig. 1 zeigt eine Schnittansicht durch eine Pille 1, die in üblicher Weise aus einer oder mehreren Wirkstoffen 2 besteht, die nach außen hin durch einen Überzug 3 bedeckt sind.

Im Inneren dieser Pille 1, d.h. also innerhalb der Wirkstoffe 2, ist nun zusätzlich noch ein erster magnetischer Körper 4 eingebettet, der aus einem in etwa serpentinenartig mehr oder weniger eng zusammengefalteten Draht oder Band oder Streifen gebildet ist, wobei dieser Draht oder dieses Band oder dieser Streifen beispielsweise aus einem amorphen Metall oder aus einer amorphen Metallverbindung besteht, d.h. also aus einem Werkstoff mit solchen magnetischen Eigenschaften, daß er in der Lage ist, auf ein von außen einwirkendes Magnetfeld zu reagieren, welches von der noch weiter unten im einzelnen zu erläuternden elektronischen Überwachungsvorrichtung erzeugt wird.

Wie die Fig. 1 weiterhin zeigt, kann der aus dem erläuterten magnetischen Werkstoff gebildete, erste magnetische Körper 4 innerhalb einer Umhüllung 5 von z. B. rechteckförmigem Querschnitt angeordnet sein, wobei diese Umhüllung 5 z. B. aus einem beim Durchgang durch den Gastro-Intestinal-Trakt eines Patienten löslichen Material, wie etwa Gelatine oder dgl. besteht.

Die Umhüllung 5 kann aber auch aus einem Material bestehen, das eine gastro-intestinal-bereichsspezifisch unterschiedliche Löslichkeit aufweist, d.h., als Ort der Auflösung ist z. B. entweder der Dünndarm oder der Dickdarm vorgegeben.

Die Umhüllung 5 kann aber auch so ausgebildet sein, daß sie nur teilweise den ersten magnetischen Körper 4 umgibt.

Aus der Fig. 2 ist eine Schnittansicht durch ein weiteres Medikament ersichtlich, das in der Form einer Kapsel 6 ausgebildet ist, bei der innerhalb einer Umhüllung 8, die z. B. aus Gelatine besteht, ein Wirkstoff oder eine Kombination von Wirksubstanzen 7 untergebracht ist. Weiterhin ist im Inneren der Kapsel 6 innerhalb der Wirksubstanzen 7 ein zweiter magnetischer Körper 9 eingebettet, der aus einem spulenförmig oder rollenförmig zusammengefügten Band oder Streifen gebildet ist, wobei dieses Band oder dieser Streifen wiederum beispielsweise aus einem amorphen Metall oder aus einer amorphen Metallverbindung besteht, d.h. aus einem Werkstoff, wie er bereits weiter oben in Verbindung mit dem ersten magnetischen Körper 4 erläutert worden ist. Der magnetische Werkstoff kann anstatt aus einem amorphen Material aber auch aus einem weichmagnetischem Material bestehen.

Es versteht sich, daß für den ersten magnetischen Körper 4 und für den zweiten magnetischen Körper 9 verschiedene magnetische Werkstoffe mit unterschiedlichen magnetischen Eigenschaften vorgesehen sein können. Hierbei sind die verschiedensten Kombinationen von Werkstoffen/Werkstoffeigenschaften mit geometrischen Formen der Körper der unterschiedlichsten Art möglich. Sowohl dem ersten magnetischen Körper 4 innerhalb der Pille 1 als auch dem zweiten magnetischen Körper 9 innerhalb der Kapsel 6 ist jedoch gemeinsam, daß diese Körper nach der Auflösung des applizierten Medikamentes im Gastro-Intestinal-Trakt eines Patienten in einen solchen geöffneten Ausgangszustand übergeführt oder zurückgeführt werden, in welchem die genannten Körper 4 bzw. 9 wiederum die Gestalt eines ausgestreckten Drahtes oder Streifens oder Bandes oder Filmes annehmen, aus dem sie ursprünglich hergestellt sind, so daß die genannten Körper erst in diesem magnetisch detektierbaren Zustand oder in dieser magnetisch detektierbaren Konfiguration die erwünschten magnetischen Eigenschaften, insbesondere Resonanzeigenschaften zeigen, wodurch die Detektierbarkeit dieser Körper mit Hilfe des von außen einwirkenden Magnetfeldes gewährleistet ist, wie dies noch weiter unten im einzelnen ausführlich erläutert wird.

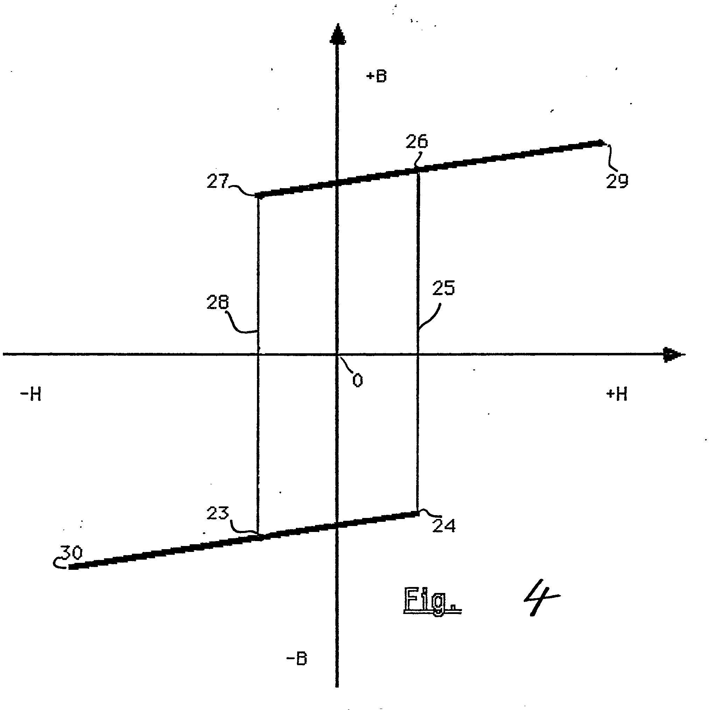

Die den einem Patienten zu verabreichenden Präparaten zugesetzten magnetischen Werkstoffe bzw. die aus diesen Werkstoffen gebildeten Körper weisen in bevorzugter Weise eine Magnetisierungskurve auf, wie sie beispielsweise in der Fig. 4 dargestellt ist. Dieses Diagramm enthält aus Gründen der Übersichtlichkeit eine vereinfacht gezeichnete Hystereseschleife mit der magnetischen Feldstärke H auf der Abszisse und der magnetischen Induktion B auf der Ordinate.

H stellt somit das von außen einwirkende magnetische Feld dar, bei dem es sich beispielsweise um ein magnetisches Wechselfeld handeln kann.

Der negative maximale Induktionspunkt ($- B_{max}$) ist mit 23, der positive Schwellwert des magnetischen Feldes mit 24, der positive maximale Induktionspunkt ($+ B_{max}$) mit 26 und der negative Schwellwert des magnetischen Feldes mit 27 bezeichnet. Wenn das magnetische Feld den Punkt 24 erreicht und diesen überschreitet, kommt es zu einer abrupten regenerativen Umkehr der Polarität, so daß auf der Hystereseschleife der Punkt 26 ($+ B_{max}$) erreicht wird. Die Linie 25 zwischen den Punkten 24 und 26 stellt somit einen großen Barkhausen-Sprung dar, gemäß dem die Polaritätsumkehr erfolgt.

Wenn auf der anderen Seite das magnetische Feld H in umgekehrter Richtung den Nullpunkt durchläuft und weiter in negativer Richtung geht, dann wird schließlich der Punkt 27, d.h. der negative Schwellwert des magnetischen Feldes erreicht, von dem aus wiederum eine abrupte regenerative Umkehr der Polarität stattfindet, bis der Punkt 23 ($- B_{max}$) erreicht ist. Die Linie 28 zwischen den Punkten 27 und 23 stellt wiederum den erneuten großen Barkhausen-Sprung in diesem Bereich dar.

Auf der Magnetisierungskurve gemäß Fig. 4 sind ferner die Punkte positiver Sättigung bzw. negativer Sättigung mit 29 bzw. 30 bezeichnet.

Selbstverständlich ist die für das jeweilige magnetische Material kennzeichnende Hystereseschleife abhängig von der speziellen Werkstoffzusammensetzung und den jeweiligen geometrischen Formen und Abmessungen des hieraus gebildeten Körpers.

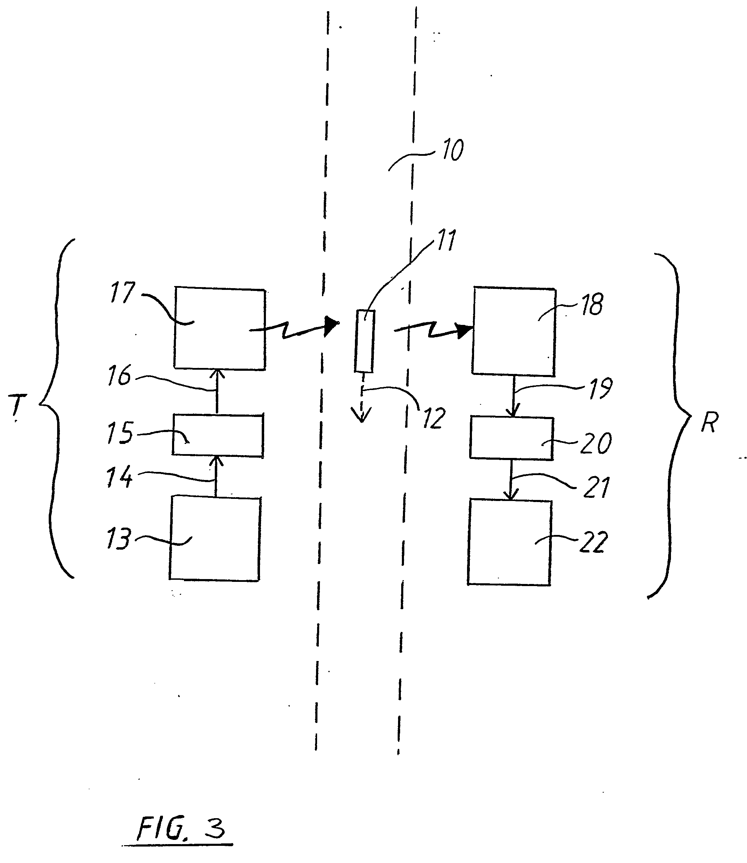

Ein bevorzugtes Ausführungsbeispiel einer elektronischen Überwachungsvorrichtung zum Überwachen, Kontrollieren, Registrieren und Anzeigen der Einnahme von pharmazeutischen Präparaten, insbesondere in oralen oder analen Applikationsformen, seitens eines Patienten, geht aus der Fig. 3 hervor, in der ein Blockschaltbild dieser Überwachungsvorrichtung dargestellt ist.

Hierbei wird zunächst vorausgesetzt, daß in Übereinstimmung mit dem Verfahren nach der vorliegenden Erfindung einem Patienten ein Präparat oder Medikament in einer vorgegebenen Dosis verabreicht worden ist und daß dieses Medikament tatsächlich von dem Patienten eingenommen worden ist.

Es handelt sich hierbei um ein Medikament, beispielsweise um eine Pille oder eine Kapsel, wie sie in den Fig. 1 oder 2 dargestellt ist, somit also um ein Medikament, dessen Wirkstoffen ein magnetischer Werkstoff bzw. ein aus diesem Werkstoff gebildeter Körper zugesetzt ist, der auf das von der Überwachungsvorrichtung gem. Fig. 3 erzeugte, äußere Magnetfeld regierfähig ist. Im übrigen kann das Präparat oder Medikament auch in jeder anderen Verabreichungsform vorliegen, beispielsweise als Pastille, Tablette, film- oder membranüberzogene Tablette oder Kapsel, Dragee, Zäpfchen oder dgl.

Es wird nun ferner von der Voraussetzung ausgegangen, daß das von dem Patienten eingenommene Medikament sich bereits innerhalb dessen Gastro-Intestinal-Traktes (GI-Traktes) aufgelöst hat, derart, daß der in diesem Medikament enthaltene, aus dem magnetischen Werkstoff gebildete Körper in einen magnetisch detektierbaren Zustand übergeführt ist, in welchem die magnetischen Eigenschaften, z. B. die Resonanzeigenschaften des magnetischen Werkstoffes, voll zur Geltung kommen.

In Fig. 3 ist der schematisch dargestellte GI-Trakt des Patienten mit 10 bezeichnet, der von einem aus dem genannten magnetischen Werkstoff bestehenden Körper 11 in dessen detektierbarem Zustand durchwandert wird, wobei die Bewegungsrichtung des Körpers 11 innerhalb des GI-Traktes 10 schematisch durch einen Pfeil 12 angedeutet ist.

Um nun zu kontrollieren oder festzustellen, ob das applizierte Präparat tatsächlich von dem Patienten eingenommen worden ist, wird durch die elektronische Überwachungsvorrichtung gemäß Fig. 3 ein äußeres Magnetfeld erzeugt, dem der GI-Trakt 10 des Patienten nunmehr ausgesetzt wird, d.h., dieser GI-Trakt 10 bildet einen Überwachungs- oder Kontrollbereich bezüglich der elektronischen Überwachungsvorrichtung. Diese Überwachungsvorrichtung weist sowohl ein Sendeteil T als auch ein Empfangsteil R auf. Das Sendeteil T besteht im wesentlichen aus einem Frequenzgenerator 13, dessen Ausgang über eine Leitung 14 mit einer Dämpfungsvorrichtung 15 verbunden ist, die vorzugsweise einstellbar ist.

Durch diese Dämpfungsvorrichtung 15 wird das von dem Frequenzgenerator 13 erzeugte Ausgangssignal mit einem gewünschten Pegel über eine weitere Leitung 16 an eine nachfolgende Felderzeugungsspule 17 gegeben, durch welche nunmehr ein magnetisches Wechselfeld aufgebaut wird, dem der GI-Trakt 10 des Patienten ausgesetzt wird. Der Empfangsteil R der Überwachungsvorrichtung gemäß Fig. 3 besteht im wesentlichen aus einer Feldempfangsspule 18, deren Ausgang über eine Leitung 19 mit einem Empfänger 20 verbunden ist, von dem aus noch eine Leitung 21 zu einem nachgeschalteten Anzeigegerät 22 führt.

Wenn nun davon ausgegangen wird, daß der Körper 11 aus einem magnetischen Werkstoff gebildet ist, dessen Magnetisierungskurve eine große Barkhausen-Unstetigkeit aufweist, die durch bedeutende sprunghafte Wechsel oder durch eine regenerative Umkehr der magnetischen Polarisation gekennzeichnet ist, wenn dieser Körper 11 in das von außen einwirkende magnetische Wechselfeld eingebracht und den positiven und negativen Amplituten dieses Feldes ausgesetzt wird, dann äußert sich das Ansprechverhalten des Körpers 11 in seinem detektierbaren Zustand auf die erwähnten sprunghaften Umkehrungen der Polarität insbesondere in der Erzeugung von starken harmonischen Schwingungen, die leicht von dem Ansprechverhalten anderer magnetischer Objekte unterschieden werden können, die sich möglicherweise ebenfalls innerhalb der Reichweite des durch die Überwachungsvorrichtung gemäß Fig. 3 erzeugten Magnetfeldes befinden können. Es werden somit durch den im GI-Trakt 10 des Patienten vorhandenen, aus dem eingenommenen Präparat stammenden magnetischen Körper 11 in dessen magnetisch detektierbarem Zustand in dem durch die Felderzeugungsspule 17 erzeugten äußeren Magnetfeld Störungen induziert, die durch die Feldempfangsspule 18 in Form von starken harmonischen Schwingungen erfaßbar sind, derart, daß ein entsprechendes Signal über die Leitung 19 zu dem Empfänger 20 geleitet wird, der vorzugsweise entsprechende Signalverarbeitungsmittel beinhaltet, so daß durch diesen Empfänger 20 selektiv detektierbare Signale erzeugt und zur Lieferung einer eindeutigen Information weiterverarbeitet werden, ob das Präparat von dem Patienten eingenommen ist oder nicht. Da gemäß dem Ausführungsbeispiel nach Fig. 3 das Präparat oder das Medikament tatsächlich eingenommen worden ist, wie bereits weiter oben erläutert, gibt der Empfänger 20 sodann ein entsprechendes Ausgangssignal über die Leitung 21 an das Anzeigegerät 22, durch das also angezeigt oder registriert wird, daß das verabreichte Präparat von dem Patienten tatsächlich eingenommen ist. Das Fehlen eines solchen Ausgangssignals stellt eine Negativ-Information dar, d.h. das Medikament ist vom Patienten nicht eingenommen worden, so daß in dessen GI-Trakt kein magnetischer Körper 11 existiert.

Fig. 5 zeigt ein Ausführungsbeispiel einer elektronischen Überwachungsvorrichtung in Übereinstimmung mit der vorliegenden Erfindung. Gemäß der hier gezeigten Ausführung ist die elektronische Überwachungsvorrichtung insgesamt als ein mobiles Gerät ausgebildet, insbesondere in der Form eines allgemein mit 31 bezeichneten mobilen Kontroll- und Anzeigegerätes. Bei diesem ist der durch ein gestrichelt gezeichnetes Kästchen angedeutete Sendeteil T gemäß Fig. 3 sowie der ebenfalls durch ein gestrichelt gezeichnetes Kästchen angedeutete Empfangsteil R gemäß Fig. 3 innerhalb eines Gehäuses 32 des Kontroll- und Anzeigegerätes 31 untergebracht, wobei dieses Gehäuse 32 im wesentlichen halbkugelförmig ausgebildet ist und auf einer Seite nach außen durch eine Platte 34 abgeschlossen ist, die dazu dient, das Kontroll- und Anzeigegerät an den Körper eines Patienten anzunähern, dessen GI-Trakt dem durch das Sendeteil T erzeugten äußeren Magnetfeld ausgesetzt wird oder aber auch zum unmittelbaren Berühren des Körpers des Patienten mit der Platte 34, beispielsweise zum Auflegen auf den Bauch eines im Krankenbett liegenden Patienten.

Das Gehäuse 32 des mobilen Kontroll- und Anzeigegerätes 31 ist ferner mit einem Handgriff 33 versehen, mit dessen Hilfe sich das Gerät sehr einfach handhaben läßt, wobei von einem freien Ende dieses Handgriffes 33 aus eine Netzanschlußleitung 35 abgeht, um das Gerät an das öffentliche Stromnetz anschließen zu können.

Stattdessen oder zusätzlich kann das Kontroll- und Anzeigegerät 31 aber auch eine eingebaute Stromversorgung aufweisen, die z. B. durch Batterien oder Solarzellen gebildet ist, wie dies in der Fig. 5 durch das gestrichelt gezeichnete Kästchen S angedeutet ist, wobei im vorliegenden Ausführungsbeispiel diese Stromversorgung im Handgriff 33 angeordnet ist.

Im übrigen weist der Handgriff 33 noch einen Schalter 36 zum Einschalten der Stromversorgung für das Kontroll- und Anzeigegerät 31 auf.

Weiterhin weist das Kontroll- und Anzeigegerät 31 ein Anzeigefenster 37 auf, das dazu dient, die von dem Empfangsteil R abgegebenen Ausgangssignale anzuzeigen, die eine Information darüber beinhalten, ob das Präparat von dem Patienten eingenommen ist oder nicht.

Mit Hilfe des aus der Fig. 5 ersichtlichen Kontroll- und Anzeigegerätes 31 ist es darüber hinaus auch noch möglich, Ausgangssignale zu erhalten, die eine Information bezüglich der Lokalisierung des vom Patienten eingenommenen Präparates bzw. des in diesem enthaltenen magnetischen Werkstoffes im Bereich des GI-Traktes des Patienten beinhalten, und zwar vorzugsweise dadurch, daß der anatomische Verlauf des GI-Traktes mit dem Kontroll- und Anzeigegerät 31 abgetastet wird.

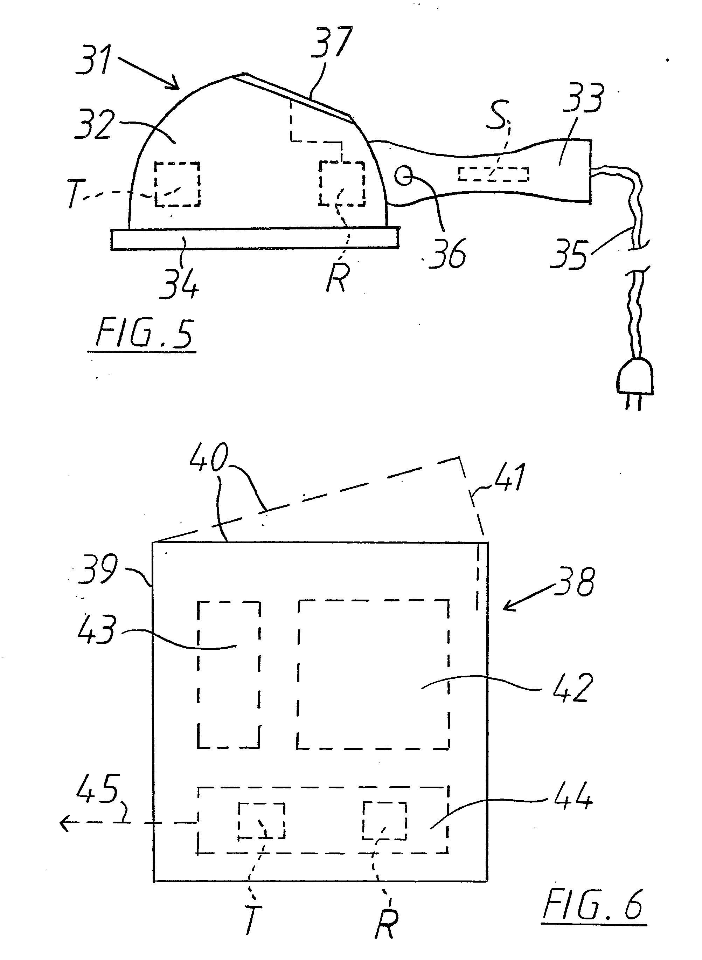

Ein weiteres Ausführungsbeispiel einer elektronischen Überwachungsvorrichtung in Übereinstimmung mit der vorliegenden Erfindung ist aus der Fig. 6 ersichtlich.

Bei dieser Ausführung handelt es sich um eine Überwachungsvorrichtung 38, die gleichzeitig als Arzneimittelverpackung ausgebildet ist. Die Überwachungsvorrichtung 38 weist hierbei ein Gehäuse 39 auf, wobei dieses Gehäuse vorzugs weise in der Form einer Schachtel ausgebildet ist, die mit einer Verschlußklappe 40 mit einer Einsteckklappe oder Lasche 41 versehen ist, um damit das Gehäuse 39 beliebig öffnen und schließen zu können, insbesondere, um durch ein Öffnen des Gehäuses 39 Zugang zu einem in diesem enthaltenen Arzneimittelbehälter 42 zu erhalten, so daß ein Patient aus diesem Arzneimittelbehälter 42 in der verordneten Dosierung und zu einem vorgeschriebenen Zeitpunkt das einzunehmende Medikament entnehmen kann.

Weiterhin ist in dem Gehäuse 39 eine Zeitregistriervorrichtung 43 untergebracht, die dazu dient, zusätzlich den jeweiligen Zeitpunkt, zu dem das Präparat oder Medikament aus dem Arzneimittelbehälter 42 durch den Patienten entnommen wird, genau zu ermitteln und zu registrieren. Diese Zeitregistriervorrichtung 43 liefert somit eine Information oder eine Aufzeichnung, gegebenenfalls auch in der Form eines entsprechenden Ausgangssignales, aus welchem sich der genaue Entnahmezeitpunkt und damit aber auch die wahrscheinliche Zeit der Dosierung ergibt. Zwar stellt der Zeitpunkt der Medikamenten-Entnahme aus dem Arzneimittelbehälter 42 lediglich eine mutmaßliche oder wahrscheinliche Zeit der Applikation oder der Einnahme des entnommenen Medikamentes dar, da es zwischen dem Zeitpunkt der Medikamenten-Entnahme und der Medikamenten-Einnahme seitens des Patienten durchaus eine Verzögerung geben kann, d.h. ein dieser Verzögerung entsprechendes Zeitintervall, jedoch kann dieses Zeitintervall nicht länger sein als dasjenige Zeitintervall, das durch die Zeit definiert ist, die zwischen der durch die Zeitregistriervorrichtung 43 dokumentierten Medikamenten-Entnahme und dem durch die elektronische Überwachungsvorrichtung gelieferten Ausgangssignal mit der Information, daß das Medikament tatsächlich vom Patienten eingenommen ist, verstrichen ist. Es ist darüber hinaus als unwahrscheinlich anzusehen, daß der Entnahme des Medikaments oder Arzneimittels aus dem Arzneimittelbehälter 42 nicht ziemlich prompt auch die Einnahme durch den Patienten folgt, falls das Medikament überhaupt eingenommen wird. Wenn jedoch durch die elektronische Überwachungsvorrichtung tatsächlich ein Ausgangssignal erzeugt und angezeigt wird, das die tatsächliche Einnahme des Medikamentes beweist, dann ist mit größtmöglicher Sicherheit die Annahme berechtigt, daß der Zeitpunkt der Medikamenten-Einnahme nur in geringem zeitlichen Abstand von denjenigem Zeitpunkt liegt, der durch die Zeitregistriervorrichtung 43 bezüglich der Medikamente-Entnahme ermittelt und registriert worden ist.

Das in der Fig. 6 dargestellte Ausführungsbeispiel der elektronischen Überwachungsvorrichtung 38 weist ferner innerhalb ihres Gehäuses 39 eine aus einem Sendeteil T und einem Empfangsteil R (vgl. Fig. 3) bestehende Einheit 44 auf. Es besteht aber auch die Möglichkeit, daß diese Einheit 44 lediglich durch das Empfangsteil R gebildet ist, während das Sendeteil T als ein separates Gerät ausgebildet ist, das an einer anderen Stelle angeordnet werden kann. Umgekehrt kann aber auch die Einheit 44 ausschließlich durch das Sendeteil T gebildet sein, während das Empfangsteil R als ein separates Gerät ausgebildet ist, das an anderer Stelle angeordnet werden kann.

Darüber hinaus besteht noch die Möglichkeit, daß eine vom Ausgang der Einheit 44 abführende Leitung 45 vorgesehen ist, über die die Einheit 44 mit einer gesonderten Anzeigeeinheit, beispielsweise mit einer an zentraler Stelle angeordneten Anzeigeeinheit verbunden ist, die im einzelnen hier nicht dargestellt ist.

Es kann sich bei der Leitung 45 aber auch um eine Antenne zur drahtlosen Übertragung der von der Einheit 44 abgegebenen Ausgangssignale zu einer zentralen Anzeigeeinheit handeln.

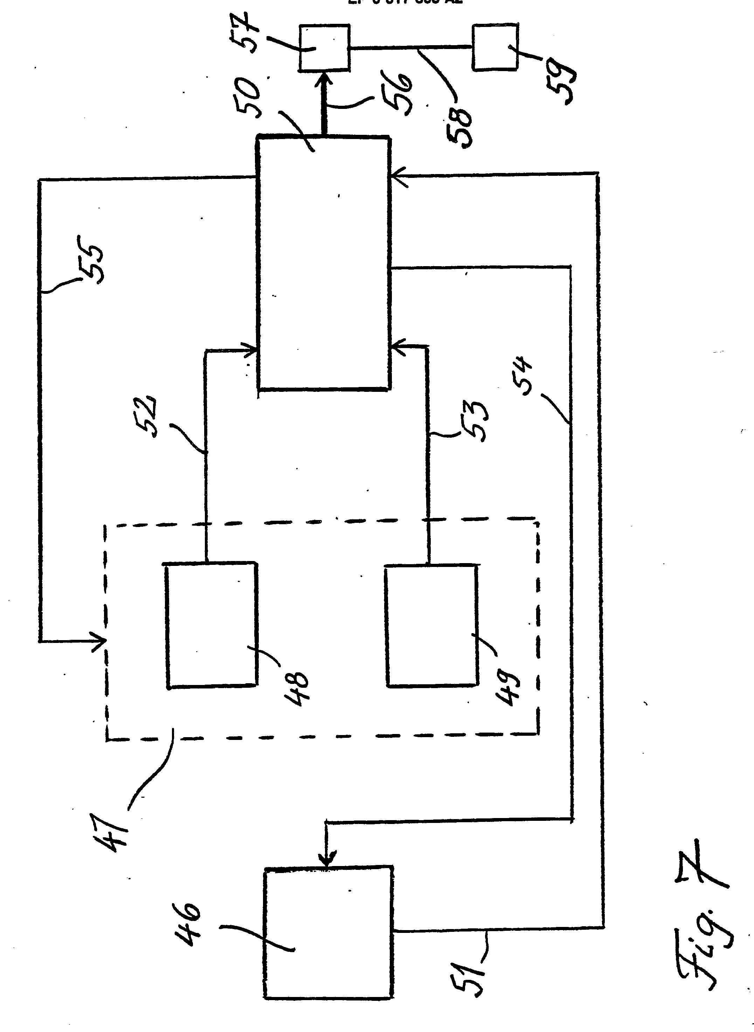

Aus Fig. 7 ist schließlich noch ein Blockschaltbild eines Systems zum Überwachen, Kontrollieren, Registrieren und Anzeigen der Einnahme von pharmazeutischen Präparaten, insbesondere in oralen oder analen Applikationsformen, seitens eines Patienten dargestellt, wobei dieses Überwachungssystem noch durch eine Einrichtung zum Ermitteln und Registrieren des jeweiligen Zeitpunktes, zu dem das Präparat aus der zugehörigen Verpackung genommen wird, sowie durch eine gesonderte Überwachungseinrichtung zur Anwesenheits- oder Identitätskontrolle des betreffenden Patienten ergänzt ist. Außerdem ist dieses Überwachungssystem mit einer zentralen Kontroll- und Steuereinheit verbunden.

Das aus der Fig. 7 ersichtliche System weist somit ein Zeit-Kontroll- und Registriergerät 46 auf, das über eine Leitung 51 mit einer zentralen Kontroll- und Steuereinheit 50 verbunden ist. Ferner weist das Überwachungssystem ein Gerät 48 zur Kontrolle der Medikamenten-Einnahme seitens eines Patienten sowie ein Gerät 49 zur Anwesenheits-und/oder Identitätskontrolle des betreffenden Patienten auf, wobei diese beiden Geräte 48 und 49 zu einer Überwachungseinheit 47 zusammengefaßt sind. Das Gerät 48 zur Medikamenten-Einnahme-Kontrolle ist über eine Leitung 52 und das Gerät 49 zur Anwesenheits-/Identitäts-Kontrolle ist über eine Leitung 53 mit der zentralen Kontroll- und Steuereinheit 50 verbunden.

Auf der anderen Seite führt von der zentralen Kontroll- und Steuereinheit 50 aus eine weitere Leitung 54 zu dem Zeit-Kontroll- und Registriergerät 46 und eine weitere Leitung 55 zu der Überwachungseinheit 47.

Von der zentralen Kontroll- und Steuereinheit 50 aus führt schließlich ein Bus 56 zu einem Mikro-Computer 57, an den über eine Leitung 58 ein Drucker 59 angeschlossen ist.

Das in Fig. 7 dargestellte Überwachungssystem funktioniert im einzelnen so, daß zunächst von dem Zeit-Kontroll- und Registriergerät 46 ein Ausgangssignal abgegeben wird, das eine Information bezüglich des Zeitpunktes der Entnahme eines Medikaments aus der zugehörigen Verpackung beinhaltet, wobei dieses Ausgangssignal über die Leitung 51 zur zentralen Kontroll- und Steuereinheit geschickt und dort aufgezeichnet wird. Sodann wird innerhalb eines vorgegebenen Zeitintervalles nach dem von dem Zeit-Kontroll- und Registriergerät 46 gelieferten Zeitpunkt mit Hilfe des Gerätes 48 zur Medikamenten-Einnahme-Kontrolle festgestellt, ob das aus der Arzneimittelverpackung entnommene Medikament tatsächlich von dem Patienten eingenommen ist oder nicht, wobei von dem Gerät 48 entsprechende Ausgangssignale erzeugt werden, die über die Leitung 52 zur zentralen Kontroll- und Steuereinheit 50 übertragen werden.

Bei dem Gerät 48 handelt es sich beispielsweise um eine Überwachungsvorrichtung, deren Komponenten in der Fig. 3 im einzelnen dargestellt sind.

Zum gleichen Zeitpunkt, zu dem die Medikamenten-Einnahme-Kontrolle mit Hilfe des Gerätes 48 durchgeführt wird, oder aber zu einem diesem Zeitpunkt sehr naheliegenden Zeitpunkt vorher oder nachher bzw. innerhalb desselben vorgegebenen Zeitintervalles wird mit Hilfe des Gerätes 49 innerhalb der Überwachungseinheit 47 festgestellt, ob der betreffende Patient überhaupt anwesend war und der Kontrolle mit Hilfe des Gerätes 48 unterzogen wurde, eventuell auch mit gleichzeitiger Identifizierung des Patienten.

Ein entsprechendes Ausgangssignal, welches die Information beinhaltet, ob der betreffende Patient tatsächlich anwesend war oder nicht, wird von dem Gerät 49 aus über die Leitung 53 ebenfalls zur zentralen Kontroll- und Steuereinheit 50 geschickt.

Bei dem Gerät 49 zur Anwesenheits-/Identitäts-Kontrolle kann es sich im übrigen um eine elektronische Überwachungsvorrichtung handeln, die in gleicher oder ähnlicher Weise ausgebildet ist wie die elektronische Überwachungsvorrichtung in Übereinstimmung mit der vorliegenden Erfindung, andererseits besteht aber auch die Möglichkeit, die beiden Geräte 48 und 49 zu einem einzigen Gerät in Form einer kombinierten elektronischen Überwachungsvorrichtung zu vereinigen, die sowohl zur Medikamenten-Einnahme-Kontrolle als auch zur Patienten-Anwesenheits-Kontrolle dient.

Somit kann das Gerät 49 zur Anwesenheits-/Identitäts-Kontrolle insbesondere in der Form einer elektronischen Überwachungsvorrichtung ausgebildet sein, durch die ein äußeres Magnetfeld, beispielsweise in der Form eines magnetischen Wechselfeldes erzeugt wird, während der zu kontrollierende Patient an oder in seinem Körper oder an oder in seiner Kleidung einen magnetischen Werkstoff oder ein aus diesem magnetischen Werkstoff gebildeten Körper trägt, der auf das genannte äußere Magnetfeld der Überwachungsvorrichtung reagierfähig ist, um ein durch diese Überwachungsvorrichtung selektiv detektierbares Signal zur Lieferung einer eindeutigen Information abzugeben, ob der betreffende Patient anwesend war oder nicht. Hierbei kann z. B. das magnetische Material oder der aus diesem gebildete magnetische Körper auch an oder in anderen Gegenständen untergebracht sein, die von dem betreffenden Patienten an seinem Körper getragen werden, beispielsweise eine Armbanduhr, ein Ring oder andere Schmuckstücke oder dgl. mehr. Darüber hinaus kann aber auch das magnetische Material oder der aus diesem gebildete magnetische Körper in der Form eines Implantates ausgebildet sein, das in den Körper des betreffenden Patienten implantiert ist.

Durch geeignete Auswahl der magnetischen Werkstoffe für die Zwecke der Patienten-Anwesenheits-/Identitäts-Kontrolle kann von vornherein sichergestellt werden, daß es sich bei den durch das Gerät 49 selektiv zu detektierenden Signalen um grundsätzlich andersgeartete Signale als diejenigen Signale handelt, die von dem Gerät 48 zur Medikamenten-Einnahme-Kontrolle erzeugt werden, so daß die Gefahr von Interferenzen oder der Verwechslung der durch die beiden Geräte 48 und 49 erzeugten Ausgangssignale von vornerein gebannt ist.

Das Gerät 49 zur Anwesenheits-Kontrolle kann aber auch funktionsmäßig auf anderen Prinzipien zur Feststellung der menschlichen Anwesenheit beruhen, beispielsweise auf der Grundlage von thermischen Messungen, einer Detektion der Sprache oder der Stimme, auf der Grundlage von $CO_2$-Messungen, von irgendwelchen anderen akustischen Messungen oder auf der Grundlage einer Kombination der vorgenannten Detektions-Maßnahmen.

Von der zentralen Kontroll- und Steuereinheit 50 aus können sowohl über die Leitung 54 als auch über die Leitung 55 Steuersignale einerseits zu dem Zeit-Kontroll- und Registriergerät 46 und andererseits zu der Überwachungseinheit 47 geschickt werden, derart, daß eine zeitliche Steuerung der Inbetriebnahme und der Funktion der verschiedenen Geräte 46, 48 und 49 gewährleistet ist.

Schließlich kann noch vorgesehen sein, daß sämtliche Informationen und Daten, die in der zentralen Kontroll- und Steuereinheit 50 empfangen werden, durch den an diese Einheit angeschlossenen Mikrocomputer 57 weiterverarbeitet und gespeichert und anschließend durch den Drucker 59 ausgedruckt werden. Die zentrale Kontroll- und Steuereinheit 50 kann insbesondere in einer Medikamenten-Ausgabe-Zentrale einer medizinischen Klinik oder dgl. installiert sein, so daß dort sämtliche von dem Überwachungssystem gelieferten Informationen erfaßt und/oder von dort aus sämtliche Funktionen des Überwachungssystems gesteuert werden können.

Bezugszeichenliste

1 Pille
2 Wirkstoff
3 Überzug
4 Erster magnetischer Körper
5 Umhüllung
6 Kapsel
7 Wirkstoff
8 Umhüllung (von 7)
9 Zweiter magnetischer Körper
10 Gastro-Intestinal-Trakt
11 Körper aus magnetischem Werkstoff im detektierbaren Zustand
12 Bewegungsrichtung des Körpers 11
13 Frequenzgenerator
14 Leitung
15 Dämpfungsvorrichtung
16 Leitung
17 Felderzeugungsspule
18 Feldempfangsspule
19 Leitung
20 Empfänger
21 Leitung
22 Anzeigegerät
23 Negativer maximaler Induktionspunkt
24 Positiver Schwellwert des magnetischen Feldes
25 Barkhausen-Sprung
26 Positiver maximaler Induktionspunkt
27 Negativer Schwellwert des magnetischen Feldes
28 Barkhausen-Sprung
29 Punkt positiver Sättigung
30 Punkt negativer Sättigung
31 Kontroll- und Anzeigegerät
32 Gehäuse (von 31)
33 Handgriff
34 Platte
35 Netzanschlußleitung

36 Schalter
37 Anzeigefenster
38 Überwachungsvorrichtung
39 Gehäuse oder Schachtel (von 38)
40 Verschlußklappe
41 Einsteckklappe oder -lasche
42 Arzneimittelbehälter
43 Zeitregistriervorrichtung
44 Einheit aus Sende- und Empfangsteilen
45 Leitung
46 Zeit-Kontroll- und Registriergerät
47 Überwachungseinheit
48 Gerät zur Einnahme-Kontrolle
49 Gerät zur Anwesenheits-Kontrolle
50 Zentrale Kontroll- und Steuereinheit
51 Leitung
52 Leitung
53 Leitung
54 Leitung
55 Leitung
56 Bus
57 Mikrocomputer
58 Leitung
59 Drucker
T Sendeteil
R Empfangsteil
S eingebaute Stromversorgung

**Ansprüche**

1. Verfahren zum Überwachen und/oder Kontrollieren und/oder Registrieren und/oder Anzeigen der Einnahme von pharmazeutischen Präparaten (Arzneimitteln), insbesondere in oralen oder analen Applikationsformen, seitens eines Patienten, gekennzeichnet durch die Kombination der folgenden Merkmale:

a) Dem Patienten wird ein Präparat in einer vorgegebenen Dosis verabreicht, wobei diesem Präparat, insbesondere dem (den) Wirkstoff(en) des Präparats wenigstens ein magnetischer Werkstoff zugesetzt ist, der auf ein von außen einwirkendes Magnetfeld einer elektronischen Überwachungsvorrichtung reagierfähig ist;

b) es wird ein äußeres Magnetfeld erzeugt, dem der Gastro-Intestinal-Trakt des jeweiligen Patienten innerhalb mindestens eines vorgegebenen Zeitintervalles ausgesetzt wird; und

c) es werden diejenigen Störungen, die in dem erzeugten Magnetfeld durch einen oder mehrere gegebenenfalls im Gastro-Intestinal-Trakt des Patienten vorhandene, aus dem magnetischen Werkstoff bestehende Körper in derem magnetisch detektierbaren Zustand induziert werden, empfangen und/oder detektiert und/oder angezeigt, und zwar in der Weise, daß durch die elektronische Überwachungsvorrichtung selektiv detektierbare Signale erzeugt werden, die zur Lieferung einer eindeutigen Information, ob das Präparat von dem Patienten eingenommen ist oder nicht, und/oder zur Lokalisierung des Präparates bzw. des magnetischen Werkstoffes im Bereich des Gastro-Intestinal-Traktes des Patienten weiter verarbeitet werden.

2. Verfahren nach Anspruch 1,
dadurch gekennzeichnet,
daß zusätzlich jeweils der Zeitpunkt, zu dem das Präparat aus der zugehörigen Verpackung entnommen wird, erfaßt und/oder registriert und/oder angezeigt wird, insbesondere durch entsprechende Signal-Erzeugung und -Verarbeitung.

3. Verfahren nach Anspruch 1 oder 2,
dadurch gekennzeichnet,
daß das äußere Magnetfeld der elektronischen Überwachungsvorrichtung in der Form eines magnetischen Wechselfeldes erzeugt wird.

4. Verfahren nach einem der Ansprüche 1 - 3,
dadurch gekennzeichnet,
daß der Gastro-Intestinal-Trakt des jeweiligen Patienten in periodisch aufeinander folgenden Zeitintervallen bzw. zu periodisch aufeinander folgenden Zeitpunkten dem von der elektrischen Überwachungsvorrichtung erzeugten äußeren Magnetfeld ausgesetzt wird.

5. Verfahren nach einem der vorangehenden Ansprüche,
dadurch gekennzeichnet,
daß zu dem gleichen Zeitpunkt, zu dem der Gastro-Intestinal-Trakt des jeweiligen Patienten dem von der elektronischen Überwachungsvorrichtung erzeugten äußeren Magnetfeld ausgesetzt wird, oder zu einem diesem Zeitpunkt sehr nahe liegenden Zeitpunkt bzw. innerhalb des gleichen vorgegebenen Zeitintervalles eine Anwesenheits- und/oder Identitäts-Kontrolle dieses Patienten durchgeführt wird, und zwar entweder mit Hilfe der gleichen elektronischen Überwachungsvorrichtung oder mit Hilfe einer zusätzlichen, ausschließlich zur Anwesenheits-und/oder Identitätskontrolle dienenden Überwachungsvorrichtung, wobei entsprechende Signale erzeugt und verarbeitet werden.

6. Verfahren nach einem der vorangehenden Ansprüche,
dadurch gekennzeichnet,
daß sämtliche erzeugten Signale zu einer zentralen Kontroll-und Steuereinheit übertragen werden.

7. Vorrichtung zum Überwachen und/oder Kontrollieren und/oder Registrieren und/oder Anzeigen der Einnahme von pharmazeutischen Präparaten (Arzneimitteln), insbesondere in oralen oder analen Applikationsformen, seitens eines Patienten, zur Durchführung des Verfahrens nach Anspruch 1,

dadurch gekennzeichnet,

daß diese Vorrichtung als eine elektronische Überwachungsvorrichtung ausgebildet ist, die im wesentlichen die folgenden Bestandteile aufweist:

a) Mittel zum Erzeugen eines äußeren Magnetfeldes, dem der Gastro-Intestinal-Trakt des jeweiligen Patienten innerhalb mindestens eines vorgegebenen Zeitintervalles ausgesetzt wird;

b) Mittel zum Empfangen und/oder selektiven Detektieren von Störungen, die in dem erzeugten Magnetfeld durch einen oder mehrere gegebenenfalls im Gastro-Intestinal-Trakt des Patienten vorhandene, aus einem magnetischen, auf das von außen einwirkende Magnetfeld reagierfähigen Werkstoff bestehende Körper in derem magnetisch detektierbaren Zustand induziert werden;

c) Mittel zum Erzeugen von selektiv detektierbaren Signalen aufgrund der empfangenen und/oder selektiv detektierten Störungen in dem erzeugten Magnetfeld;

d) Mittel zur Weiterverarbeitung der gemäß c) erzeugten Signale zur Lieferung einer eindeutigen Information, ob das Präparat von dem Patienten eingenommen ist oder nicht und/oder zur Lokalisierung des Präparates bzw. des magnetischen Werkstoffes im Bereich des Gastro-Intestinal-Traktes des Patienten, in Form entsprechender Ausgangssignale; und

e) Mittel zum Registrieren und/oder Anzeigen der gemäß d) gebildeten Ausgangssignale.

8. Vorrichtung nach Anspruch 7, zur Durchführung des Verfahrens nach Anspruch 2, dadurch gekennzeichnet, daß die elektronische Überwachungsvorrichtung zusätzlich Mittel zum Erfassen und/oder Registrieren und/oder Anzeigen des jeweiligen Zeitpunktes aufweist, zu dem das Präparat aus der zugehörigen Verpackung entnommen wird.

9. Vorrichtung nach Anspruch 7 oder 8, zur Durchführung des Verfahrens nach Anspruch 5, dadurch gekennzeichnet, daß die elektronische Überwachungsvorrichtung zusätzlich Mittel zum Kontrollieren (Erfassen und/oder Registrieren und/oder Anzeigen) der Anwesenheit und/oder der Identität des betreffenden Patienten aufweist, dessen Gastro-Intestinal-Trakt innerhalb mindestens eines vorgegebenen Zeitintervalles dem von der elektronischen Überwachungsvorrichtung erzeugten äußeren Magnetfeld ausgesetzt wird.

10. Vorrichtung nach Anspruch 7 oder 8, zur Durchführung des Verfahrens nach Anspruch 5, dadurch gekennzeichnet, daß die elektronische Überwachungsvorrichtung zusätzlich mit einer gesonderten Überwachungsvorrichtung kombiniert ist, die ausschließlich zum Kontrollieren (Erfassen und/oder Registrieren und/oder Anzeigen) der Anwesenheit und/oder der Identätit des betreffenden Patienten dient, dessen Gastro-Intestinal-Trakt innerhalb mindestens eines vorgegebenen Zeitintervalles dem von der elektronischen Überwachungsvorrichtung erzeugten äußeren Magnetfeld ausgesetzt wird.

11. Vorrichtung nach einem der Ansprüche 7 - 10, dadurch gekennzeichnet, daß die Mittel zur Erzeugung eines äußeren Magnetfeldes im wesentlichen aus einem Frequenzgenerator (13) und einer diesem nachgeschalteten Felderzeugungsspule (17) bestehen, daß die Mittel zum Empfangen und/oder selektiven Detektieren der in dem erzeugten Magnetfeld induzierten Störungen im wesentlichen aus einer der Felderzeugungsspule (17) gegenüber liegend angeordneten Empfängerspule (18) bestehen, daß die Mittel zur Erzeugung selektiv detektierbarer Signale im wesentlichen aus einem der Empfängerspule (18) nachgeschalteten Empfänger (20) mit integrierten Signalverarbeitungsmitteln bestehen, und daß die Mittel zum Anzeigen der erzeugten Ausgangssignale aus einem dem Empfänger (20) nachgeschalteten Anzeigegerät (22) bestehen.

12. Vorrichtung nach einem der Ansprüche 7 - 11, dadurch gekennzeichnet, daß die elektronische Überwachungsvorrichtung insgesamt als ein stationäres Gerät ausgebildet ist.

13. Vorrichtung nach einem der Ansprüche 7 - 11, dadurch gekennzeichnet, daß die elektronische Überwachungsvorrichtung insgesamt als ein mobiles Gerät ausgebildet ist.

14. Vorrichtung nach Anspruch 13, dadurch gekennzeichnet, daß die Sende- und Empfangsteile der elektronischen Überwachungsvorrichtung innerhalb eines mobilen Kontroll- und Anzeigegerätes (31) untergebracht sind, das nach außen durch eine Platte (34) abgeschlossen ist, zum Annähern an den Körper oder Berühren des Körpers des Patienten, dessen Gastro-Intestinal-Trakt dem erzeugten äußeren Magnetfeld ausgesetzt wird.

15. Vorrichtung nach Anspruch 14, dadurch gekennzeichnet, daß das mobile Kontroll- und Anzeigegerät (31) ein Anzeigefenster (37) aufweist, zur Anzeige der Information, ob das Präparat von dem Patienten eingenommen ist oder nicht und/oder zur Lokalisierungsinfo Anzeigefenster (37) aufweist, zur Anzeige der Information, ob das Präparat von dem Patienten eingenommen ist oder nicht und/oder zur Lokalisierungsinformation des Präparates bzw. des magnetischen Werkstoffes im Bereich des Gastro-Intestinal-Traktes des Patienten.

16. Vorrichtung nach Anspruch 14 oder 15,
dadurch gekennzeichnet,
daß das mobile Kontroll- und Anzeigegerät (31)
eine Netzanschlußleitung (35) zum Anschließen an
das öffentliche Stromnetz oder eine eingebaute
Stromversorgung aufweist, wie z. B. Batterie, Solarzelle oder dgl..

17. Vorrichtung nach einem der Ansprüche 7 -
16,
dadurch gekennzeichnet,
daß die elektronische Überwachungsvorrichtung
(38) gleichzeitig als Arzneimittelverpackung ausgebildet ist.

18. Vorrichtung nach Anspruch 17,
dadurch gekennzeichnet,
daß innerhalb eines Gehäuses (39) der Überwachungsvorrichtung (38) außer einer aus Sende-
und/oder Empfangsteilen bestehenden Einheit (44)
ein Arzneimittelbehälter (42) sowie eine Zeitüberwachungsvorrichtung (43) angeordnet sind, zum
zusätzlichen Erfassen und/oder Registrieren
und/oder Anzeigen des jeweiligen Zeitpunktes, zu
dem das Präparat aus dem Arzneimittelbehälter
(42) entnommen wird.

19. Vorrichtung nach einem der Ansprüche 7 -
18, zur Durchführung des Verfahrens nach Anspruch 6,
dadurch gekennzeichnet,
daß die Vorrichtung zusätzlich Mittel zur leitungsgebundenen oder drahtlosen Übertragung der erzeugten Ausgangssignale zu einer zentral angeordneten Kontroll- und Steuereinheit (50) aufweist.

20. Vorrichtung nach einem der Ansprüche 7 -
19,
gekennzeichnet durch
die Kombination folgender Merkmale:

a) Mittel (46) zum Erfassen des jeweiligen
Zeitpunktes, zu dem das Präparat aus der zugehörigen Verpackung entnommen wird;

b) Mittel (48) zur Lieferung einer Information
mittels eines äußeren Magnetfeldes, ob das Präparat von dem Patienten eingenommen ist oder nicht
und/oder zur Lokalisierung des Präparates bzw.
des in diesem enthaltenen magnetischen Werkstoffes im Bereich des Gastro-Intestinal-Traktes des
Patienten;

c) Mittel (49) zum Erfassen der Anwesenheit
und/oder der Identität des Patienten, dessen
Gastro-Intestinal-Trakt innerhalb eines vorgegebenen Zeitintervalles dem von den Mitteln (48) erzeugten äußeren Magnetfeld ausgesetzt wird;
und

d) eine zentrale Kontroll- und Steuereinheit
(50), zu der die von den Mitteln (46, 48 und 49)
erzeugten Ausgangssignale übertragen werden, zur
dortigen Registrierung und/oder Anzeige der in den
empfangenen Ausgangssignalen enthaltenen Informationen, wobei vorzugsweise die Funktionen der genannten Mittel (46, 48 und 49) von dieser zentralen Kontroll- und Steuereinheit (50) aus steuerbar
sind.

21. Vorrichtung nach Anspruch 20,
dadurch gekennzeichnet,
daß die zentrale Kontroll- und Steuereinheit (50)
über einen Bus (56) an einen Mikrocomputer (57)
angeschlossen ist, durch den sämtliche erfaßten
Daten weiterverarbeitet, gespeichert und gegebenenfalls durch einen Drucker (59) ausgedruckt werden.

Fig. 1

Fig. 2

FIG. 3

Fig. 4

31

37

32

S

33

T

34

R

36

35

_FIG.5_

40

41

39

38

43

42

45

44

T

R

_FIG.6_

Fig. 7

EP 0 317 893 A2